# EUROPEAN PATENT APPLICATION

(11) **EP 1 413 263 A1**
(43) Date of publication of application: **28.04.2004**
(21) Application number: 03256632.5
(22) Date of filing: 21.10.2003
(51) Int. Cl.: A61F 2/36, A61F 2/38

(54) **Prosthetic implant**

(30) Priority: 22.10.2002 US 278041
(71) Applicant: Zimmer Technology, Inc., Chicago, Illinois 60606 (US)
(72) Inventor: Albertorio, Ricardo, Gainesville, FL 32606-6792 (US)
(74) Representative: Mays, Julie

(57) **Abstract**

An implant (2) is provided for replacing the articular portion of a bone (72). The implant (2) includes spaces adjacent bones in articulating relation and threadably anchors the implant (2) to a bone (90). The threaded anchor (8) permits bone growth axially and transversely therethrough.

## Description

### FIELD OF THE INVENTION

The present invention relates to implants for replacing the articulating portion of a bone.

### BRIEF DESCRIPTION OF THE DRAWINGS

FIG. 1 is an exploded front plan view of an embodiment of the present invention.
FIG. 2 is a side view of the embodiment of FIG. 1.
FIG. 3 is a front plan sectional view of an embodiment of the present invention.
FIG. 4 is a front plan view of an embodiment of the present invention.
FIG. 5 is a front plan view of an embodiment of the present invention.
FIG. 6 is a partial sectional view of an embodiment of the present invention.
FIG. 7 is a front plan view of an embodiment of the present invention.

### DESCRIPTION OF THE ILLUSTRATED EMBODIMENTS

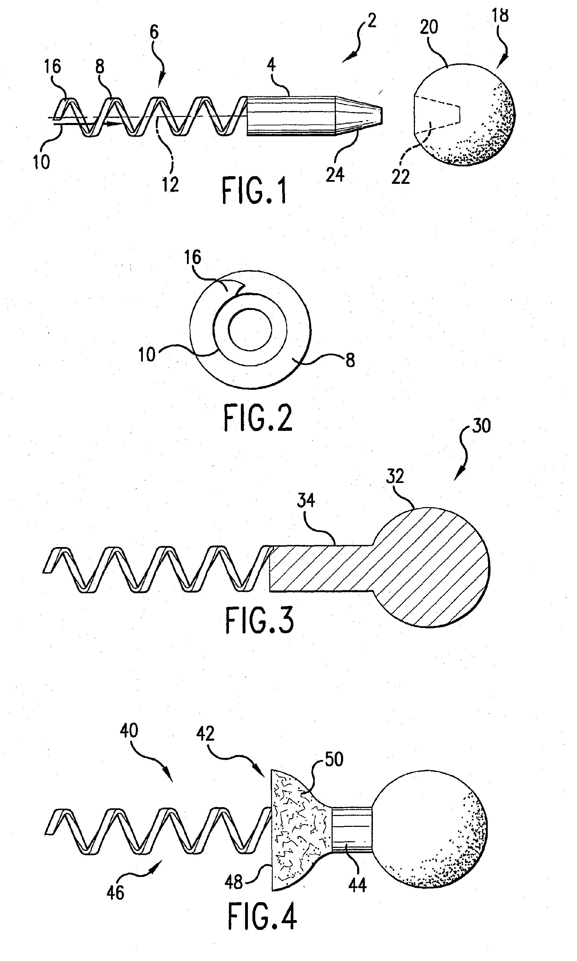

FIGS. 1-7 show several illustrative embodiments of an implant for replacing the articulating portion of a bone. The implant includes means for spacing adjacent bones in articulating relationship and means for anchoring the implant to a bone. The means for spacing adjacent bones may include an articular surface component, e.g. a neck and head or an acetabular socket as in a ball type joint such as a hip or shoulder, a tibial plateau or femoral condylar surface in a knee joint, or other spacing component for an articulating joint. The means for anchoring the implant may include a shaft extending from the spacing component such as a femoral stem component for insertion into a femoral bone, a stem for a tibial tray component for insertion into a tibial bone, or other anchoring element for another articulating joint. The implant could be made of a variety of biocompatible materials including a variety of known metals, ceramics, polymers, or other suitable materials.

Referring now to FIGS. 1 and 2, an embodiment of the invention is shown comprising a femoral implant for replacing the articular head of a femur. The implant 2 includes a neck 4 and a stem 6. The neck 4 functions to space adjacent bones in articulating relationship. The stem 6 functions to anchor the neck 4 securely to a bone. In this embodiment, the stem 6 includes a threaded portion 8 attached to the neck 4 for threadably anchoring the implant to a bone. The threaded portion 8 includes an axial bore or cannula 10 having a longitudinal axis 12. It further includes transverse openings 14. Thus the threaded portion 8 is open both axially and transversely. In particular, the threaded portion 8 of this embodiment includes an open helix. Such a stem may be formed by bending a rod around a cylindrical core, by casting, by machining from bulk material, or by using other material forming methods. The tip 16 of the threaded portion 8 may form a sharp point to ease insertion into a bone. In the particular helical embodiment shown, the helix and sharp tip have the form of a corkscrew. A modular head 18 is shown for use with the implant 2. The head 18 includes an articulating surface 20 shaped to articulate within a receiving socket of a natural or prosthetic acetabulum. The head 18 includes a neck receiving portion 22 and the neck includes a head engaging portion 24. The particular embodiment shown includes a locking taper such as a 6° taper or a 12/14 style taper as is known in the art of locking tapers. Heads 18 having varying diameters, offset distances, and offset angles may be provided. The illustrative embodiment depicts a male neck taper and a female head taper. This arrangement could be reversed within the scope of this invention. FIG. 3 shows a monoblock implant 30 similar to that of FIGS. 1 and 2 but including a head 32 that is permanently connected to the neck 34.

FIG. 4 shows another embodiment of the invention. This implant 40 is similar to the implant 2 of FIGS. 1 and 2. However, the implant 40 further includes a collar 42 intermediate the neck 44 and stem 46. The collar 42 extends outwardly beyond the stem 46 to form a bone seating surface 48 to provide further support and resistance to subsidence to the implant 40. The illustrative collar 42 shown in this embodiment projects radially to form a bone seating surface 48 completely around the neck 44 and stem 46. A partial collar is also contemplated that would only provide support for a portion of the area around the neck 44 and stem 46; e.g. a narrow collar projecting medially relative to the femoral bone. The collar in this embodiment includes a bone growth receptive surface. The bone growth receptive surface includes the bone seating surface 48 so that bone growth is received where the collar rests on the femur. The bone growth receptive surface may also include areas 50 of the collar and implant adjacent to the bone seating surface 48. The bone growth receptive surface includes materials that enhance or induce the attachment of bone to the collar, e.g. trabecular metal, beads, fiber metal, ceramics, porous ceramics, hydroxy appatite, tricalcium phosphate, plasma sprayed metal, grit blasted metal, bone growth factors, bone morphogenetic proteins, and combinations thereof. The bone growth receptive surface may be in the form of a relatively thin surface feature or the bone receptive features may extend partially or completely throughout the collar. The bone growth receptive surface may contribute to bone ongrowth, bone adhesion, bone ingrowth, or a combination thereof.

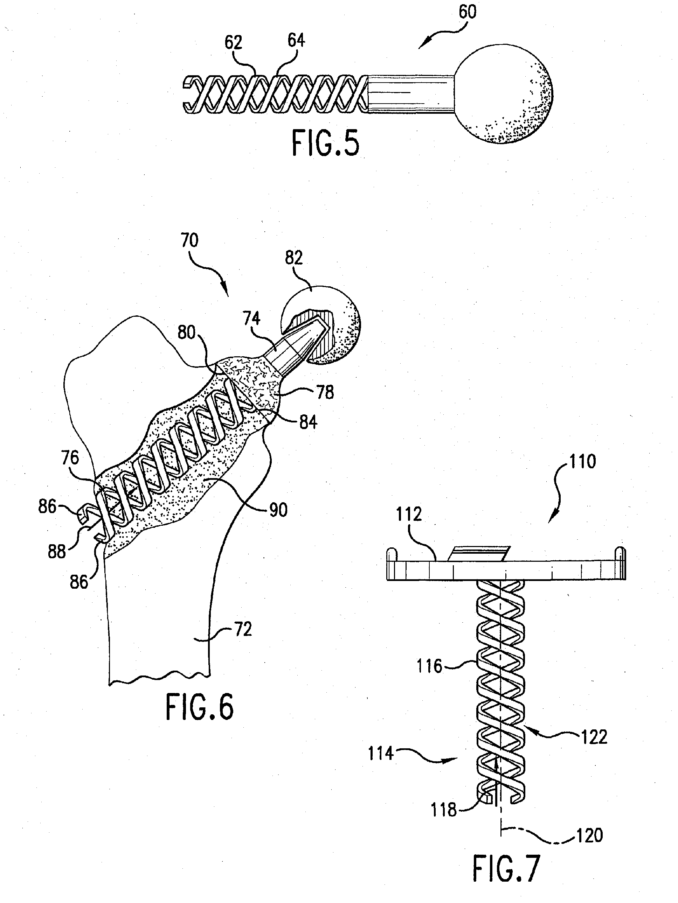

FIG. 5 depicts another embodiment of the invention. This implant 60 is similar to the implant 2 of FIGS. 1 and 2. However, the implant 60 includes first 62 and second 64 helices. Multiple helices present a relatively smooth surface that facilitates screwing the stem into a bone. Any number of helices may be provided.

FIG. 6 shows an implant 70, in situ in a human femur 72, incorporating many of the features described above. The implant 70 includes a neck 74 for spacing the adjacent bones in articulating relation and a stem 76 for anchoring the implant to the femur 72. It further includes a collar 78 intermediate the neck 74 and stem 76. The collar 78 projects radially to form a bone seating surface 80. A modular ball head 82 provides a spherical articulating surface at the end of the neck 74. The stem 76 includes a threaded portion comprising a double helix.

The implant of this invention is suitable for use in traditional open surgical approaches to a joint. It has features that make it further suitable for minimally invasive surgical approaches to articulating joints in which a smaller than normal incision is made to expose the joint. It may be difficult to prepare the bone for and insert a conventional implant through such a small incision. However, the low profile, ease of implantation, and minimal bone removal associated with the present invention makes it suitable for use through a small incision. By way of illustration, a surgical technique will be described relative to the exemplary hip joint embodiment of FIG. 6. First, a small incision is made over the hip joint and the muscles are dissected to reveal the hip joint. The natural femoral head is resected and removed leaving a boney surface 84. The implant 70 is threadably anchored on the femur 72 by screwing the stem 76 into the femur. Where the implant includes a collar 78, as in FIG. 6, the implant 70 is screwed in until the collar 78 seats on the boney surface 84. The axial and transverse openings of the threaded portion permits bone to penetrate the threaded portion to lock it in the femur. In particular, the open helical structure of the stem 76 of this embodiment is filled and surrounded, both axially and transversely, by bone 90 as it is screwed into the bone. The axial bore of the helix 88 contains a solid core of bone. The sharp tips 86 of the stem bite into the boney surface 84 and the stem 76 corkscrews into the femur without requiring prior reaming, rasping, tapping, drilling, or otherwise removing bone from the region of the bone to be occupied by the stem. It is contemplated that there may be cases where limited bone removal such as by drilling or reaming may be desirable prior to inserting an implant according to the present invention, but even in those cases, it would be much less extensive than is required for a conventional implantation and would utilize more compact instruments than conventional reamers and rasps. Where a modular head 82 is provided, it is most conveniently seated on the neck 74 after the implant is seated. The helical, open thread design of this embodiment allows good bone purchase and anatomic load transfer. Implantation causes minimal damage to the femoral canal and the cancellous bone bed. The self-tapping nature of the thread configuration allows implantation of the device with minimal instrumentation while maintaining a solid core of cancellous bone inside the thread internal diameter.

The illustrative embodiments described above are useful as proximal femoral implants for restoring the biomechanic function of the hip joint in cases of disease affecting the patient's natural femoral head, e.g. osteonecrosis, neck fracture, osteoarthritis, etc. This device may be used in traditional open surgical approaches. It is also useful for use in minimally invasive surgical techniques where its low profile, ease of implantation, and minimal bone loss during implantation are advantageous. The stem, including any helix, may also include a bone growth receptive material or structure, e.g. trabecular metal, beads, fiber metal, ceramics, porous ceramics, hydroxy appatite, tricalcium phosphate, plasma sprayed metal, grit blasted metal, bone growth factors, bone morphogenetic proteins, and combinations thereof.

FIG. 7 depicts another embodiment of the invention relating to a stemmed implant other than a femoral hip implant. In this embodiment, a tibial implant 110 is provided having a tibial tray 112 and a stem 114. The tibial tray 112 functions to space adjacent bones of the knee joint in articulating relationship. An optional, modular, articulating surface component is receivable on the tibial tray to provide a modular articulating surface. The stem 114 functions to anchor the implant 110 to the tibial bone. In the illustrative embodiment, the stem 114 includes a dual open helix 116 attached to the tibial tray 112 for threadably anchoring the implant to a bone. The helix 116 includes an axial bore or cannula 118 having a longitudinal axis 120. It further includes transverse openings 122 and is otherwise similar to the helices described in reference to the other embodiments. Bone growth receptive surfaces may be used with the tibial tray 112 and stem 114 as described with reference to the other embodiments. Implantation of the implant 110 would include screwing the stem 114 into the tibial bone to anchor the implant to the tibia. It is to be understood that the aspects of the invention described above with reference to the illustrated embodiments are applicable to a variety of implants for replacing the articulating portion of a bone in a variety of skeletal joints.

## Claims

1. A femoral implant for replacing the articular head of a femur, the implant comprising:
a neck having a ball receiving portion; and
a stem extending opposite the neck to a distal end, the stem including an open helix able to penetrate and engage the femoral bone in corkscrew fashion.

2. The implant of claim 1 wherein the helix permits bone to occupy the helix axially and transversely.

3. The implant of claim 1 wherein the stem includes a plurality of helices.

4. The implant of claim 1 further including a collar intermediate the neck and stem, the collar extending outwardly beyond the stem.

5. The implant of claim 4 wherein the collar extends radially outwardly beyond the stem to form a bone seating surface.

6. The implant of claim 4 wherein the collar has a bone growth receptive surface.

7. The implant of claim 6 wherein the bone growth receptive surface includes a material selected from the group of trabecular metal, beads, fiber metal, ceramics, porous ceramics, hydroxy appatite, tricalcium phosphate, plasma sprayed metal, grit blasted metal, bone growth factors, bone morphogenetic proteins, and combinations thereof.

8. The implant of claim 1 further including an articulating ball integrally formed with the ball receiving portion.

9. An implant comprising:
means for spacing adjacent bones in articulating relation; and
means for threadably anchoring the implant to a bone, the means for threadably anchoring the implant permitting bone growth axially transversely therethrough.

10. The implant of claim 9 wherein the means for threadably anchoring the implant to a bone comprises an open helix.

11. The implant of claim 10 wherein the means for threadably anchoring the implant to a bone includes a plurality of open helices.

12. The implant of claim 9 further including a collar intermediate the means for spacing adjacent bones for articulating relation and the means for threadably anchoring the implant to a bone, the collar extending outwardly to form a bone seating surface.

13. The implant of claim 12 wherein the collar extends radially outwardly.

14. The implant of claim 12 wherein the collar has a bone growth receptive surface.

15. The implant of claim 14 wherein the bone growth receptive surface includes a material selected from the group of trabecular metal, beads, fiber metal, ceramics, porous ceramics, hydroxy appatite, tricalcium phosphate, plasma sprayed metal, grit blasted metal, bone growth factors, bone morphogenetic proteins, and combinations thereof.

16. An implant for replacing the articulating portion of a bone, the implant comprising a body, the body having an articulating portion and an anchoring portion, the anchoring portion including an open helical anchor element engageable with said bone.

17. The implant of claim 16 wherein the helix permits bone to occupy the helix axially and transversely.

18. The implant of claim 16 wherein the anchor element includes a plurality of helices.

19. A method for replacing the articulating portion of a bone, the method comprising the steps of:
providing an implant having a bone spacing portion and a threaded anchor that permits bone growth axially and transversely therethrough;
exposing an end of the bone;
removing the natural articulating portion from the end of the bone;
threading the threaded anchor into the end of the bone; and
permitting bone to grow axially and transversely through the threaded anchor.
